# EUROPEAN PATENT APPLICATION

(11) **EP 4 563 589 A1**
(43) Date of publication of application: **04.06.2025**
(21) Application number: 23846866.4
(22) Date of filing: 10.07.2023
(51) Int. Cl.: C07K 14/00, A01N 63/50, A61K 38/00, A61P 31/04, C12N 15/70, C12R 1/19

(54) **ANTIBACTERIAL PROTEIN HAVING LYTIC ACTIVITY AGAINST STREPTOCOCCI**

(30) Priority: 26.07.2022 KR 20220092212
(71) Applicant: Intron Biotechnology, Inc., Seongnam-si, Gyeonggi-do 13202 (KR)
(72) Inventor: YOON, Seong Jun, Seoul 06281 (KR); JUN, Soo Youn, Seoul 06518 (KR); JUNG, Gi Mo, Seoul 08725 (KR); CHOI, Ji Hye, Seongnam-si Gyeonggi-do 13245 (KR); KANG, Sang Hyeon, Seoul 05501 (KR)
(74) Representative: Latscha Schöllhorn Partner AG
(86) International application number: PCT/KR2023/009728
(87) International publication number: WO 2024/025207

(57) **Abstract**

The present invention relates to an antimicrobial protein SBL2200 having antimicrobial activity specific for Streptococci and, more specifically, to: a Streptococci-specific antimicrobial protein SBL2200 having the capacity to specifically lyse Streptococci and having an amino acid sequence represented by SEQ ID NO: 1; and a pharmaceutical composition for the treatment of infections caused by Streptococci, comprising the Streptococci-specific antimicrobial protein SBL2200 as an active ingredient.

## Description

### Technical Field

The present disclosure relates to an antibacterial protein SBL2200, which has lytic activity against *Streptococci.* More specifically, the present disclosure relates to an antibacterial protein SBL2200 specific for the *Staphylococci,* the antibacterial protein SBL2200 having an ability to specifically lyse *Streptococci,* which can cause infectious diseases, and containing an amino acid sequence represented by SEQ ID NO: 1. The present disclosure also relates to a pharmaceutical composition for treating infectious diseases caused by *Streptococci,* containing the Streptococci-specific antibacterial protein SBL2200 as an active ingredient.

### Background Art

*Streptococci* are gram-positive bacteria that grow in pairs or chains. Depending on the degree of hemolysis caused by *Streptococci,* hemolysis is divided into α-hemolytic (partial hemolysis), β-hemolytic (complete hemolysis), and γ-hemolytic (no hemolysis; enterococcus). The α-hemolytic hemolysis is further classified depending on biochemical properties (optochin susceptibility). β-hemolytic hemolysis is further divided into Group (serotype) A to V depending on serological properties (Lancefield classification) of C-polysaccharide produced during metabolism.

*Streptococci* are widely distributed in nature. Some species are part of the normal human flora, but *S. pneumoniae, S. pyogenes,* and *S. agalactiae* are known to be serious pathogenic bacteria. Infections caused by these pathogenic *Streptococci* include bacteremia, endocarditis, abscess, tooth decay, pneumonia, meningitis, sinusitis, otitis media, pharyngitis, scarlet fever, pyodermatosis, cellulitis, necrotizing fasciitis, rheumatic fever, glomerulonephritis, neonatal infections (meningitis, pneumonia, and bacteremia), urethral infections, amnionitis, endometritis, and wound infections.

Specifically, the main causative agent of Group A streptococcal infections is known to be *S. pyogenes. S. pyogenes* is known to cause non-invasive infections (such as pharyngitis, pyodermatosis, and cellulitis) mainly through the throat and skin, and various invasive infections (such as rheumatic fever, acute glomerulonephritis, sepsis, scarlet fever, and bacteremia). As of 2018, tens of millions of non-invasive Group A streptococcal infections occurred in the United States. It is reported that approximately 12,500 to 20,000 people are infected with invasive Group A *Streptococci* each year, and approximately 1,250 to 1,900 of these people die. Antibiotics used to treat Group A streptococcal infections include penicillin, ampicillin, erythromycin, clindamycin, and macrolides. Among these, the antibiotic resistance rate for erythromycin, clindamycin, and macrolides is increasing by about 20% every year as of 2017.

Specifically, the main causative agent of Group B streptococcal infections is known to be *S. agalactiae. S. agalactiae* mainly lives in the intestinal tract and female genital tract, and infects newborns during delivery, causing sepsis and meningitis and causes bacteremia, pneumonia, bone and joint infections, and skin and soft tissue infections in adults. As of 2016, there were approximately 31,000 serious Group B streptococcal infections in the United States, of which, approximately 1,700 have been reported dead. To treat these Group B streptococcal infections, penicillin, clindamycin, and erythromycin are mainly used. However, it has been reported that clindamycin and erythromycin show antibiotic resistance rates of approximately 40% to 60% as of 2016.

Developing new antibacterial substances is necessary to effectively deal with infections caused by *Streptococci* that have acquired resistance to conventional antibiotics. In particular, the development of pharmaceutical agents with new mechanisms of action is urgently needed.

### Disclosure

### Technical Problem

Accordingly, as a solution to the treatment problems with the conventional antibiotics against the harmful pathogenic bacteria *Streptococci,* the present inventor(s) seek to provide an antibacterial protein SBL2200 which can specifically lyse *Streptococci.* Furthermore, the present inventor(s) further seek to provide a pharmaceutical composition, which contains this antibacterial protein as an active ingredient and can be used to treat infections caused by *Streptococci.* Furthermore, the present inventor(s) further seek to provide a method of effectively treating infections caused by *Streptococci* using the pharmaceutical composition.

Therefore, one objective of the present disclosure is to provide an antibacterial protein SBL2200 having an antibacterial activity to specifically lyse *Streptococci* and containing an amino acid sequence represented by SEQ ID NO: 1.

Another objective of the present disclosure is to provide a method of efficiently preparing an antibacterial protein SBL2200 having an antibacterial activity to specifically lyse *Streptococci* and containing an amino acid sequence represented by SEQ ID NO: 1. In this regard, *Escherichia coli* AB-22 is provided as a production strain of the antibacterial protein SBL2200. The production strain *Escherichia coli* AB-22 was deposited at the Biological Resource Center of the Korea Research Institute of Bioscience and Biotechnology (KRIBB) on July 12, 2022 (Accession No. KCTC15033BP).

A further objective of the present disclosure is to provide a pharmaceutical composition for treating infections caused by *Streptococci,* containing the antibacterial protein SBL2200 capable of specifically lysing *Streptococci* as an active ingredient.

In addition, a yet further objective of the present disclosure is to provide a method of treating infections caused by *Streptococci* by using the pharmaceutical composition containing the antibacterial protein SBL2200 as an active ingredient.

### Technical Solution

To achieve the objectives, the inventor(s) of the present disclosure utilized information on various antibacterial proteins known to have antibacterial activity against various bacterial species, and utilized their knowledge and expertise to the fullest to prepare various antibacterial protein candidates in the form of recombinant proteins and examined lytic activity of the antibacterial protein candidates against *Streptococci.* Thereby, the inventor(s) selected an antibacterial protein with excellent lytic activity and developed a method of efficiently preparing the antibacterial protein, and lastly developed a pharmaceutical composition that can be used for treating infections caused by *Streptococci* by using the antibacterial protein as an active ingredient. As a result, the inventor(s) completed the present disclosure.

Therefore, according to one aspect of the present disclosure, the present disclosure provides an amino acid sequence of an antibacterial protein SBL2200, which may specifically lyse *Streptococci.* Specifically, the amino acid sequence of the antibacterial protein SBL2200 corresponds to an amino acid sequence represented by SEQ ID NO: 1. The antibacterial protein SBL2200, which may specifically lyse *Streptococci* is made of 279 amino acid residues and has a molecular weight of approximately 31.3 kDa.

It is obvious that the amino acid sequence represented by SEQ ID NO: 1 may be partially modified by those skilled in the art using known techniques. The modifications include partial substitution of the amino acid sequence, partial addition of the amino acid sequence, and partial deletion of the amino acid sequence. However, it is most preferable to apply the amino acid sequence represented by SEQ ID NO: 1 disclosed in the present disclosure.

Additionally, the present disclosure provides a production strain *Escherichia coli* AB-22, which can be used to produce the antibacterial protein SBL2200 containing the amino acid sequence represented by SEQ ID NO: 1. The production strain *Escherichia coli* AB-22 is a strain to produce SBL2200, constructed by the present inventor(s) by transforming *E. coli* with a plasmid containing a nucleic acid sequence represented by SEQ ID NO: 2 (4, 907 bp) .

According to another aspect of the present disclosure, the present disclosure provides a pharmaceutical composition containing the antibacterial protein SBL2200 as an active ingredient for being effectively used for treating infections caused by *Streptococci,* in which the antibacterial protein SBL2200 contains the amino acid sequence represented by SEQ ID NO: 1 and has specific lytic activity against *Streptococci.*

According to the present disclosure, the antibacterial protein SBL2200, which is contained in the pharmaceutical composition of the present disclosure, contains the amino acid sequence represented by SEQ ID NO: 1 according to the present disclosure and has specific lytic activity against *Streptococci,* as described above, specifically lyses *Streptococci.* Thus, the antibacterial protein SBL2200 is effective in treating various diseases caused by *Streptococci.* Thus, the pharmaceutical composition of the present disclosure is developed as an antibiotic, a disinfectant, a sterilizer, and a therapeutic agent to treat various diseases caused by *Streptococci.*

Therefore, according to a further aspect of the present disclosure, the present disclosure provides a method of treating various diseases caused by *Streptococci* by administering the antibacterial protein SBL2200 to individuals infected with *Streptococci,* the antibacterial protein SBL2200 containing the amino acid sequence represented by SEQ ID NO: 1 and being specific for *Streptococci.*

The term "disease caused by streptococcus" in this specification refers to a disease caused by streptococcal infections. The disease includes bacteremia, endocarditis, abscess, cavities, pneumonia, meningitis, sinusitis, otitis media, pharyngitis, scarlet fever, pyodermatosis, cellulitis, necrotizing fasciitis, rheumatic fever, glomerulonephritis, neonatal infections (meningitis, pneumonia, and bacteremia), urinary tract infection, amnionitis, endometritis, or wound infections but is not limited thereto.

It does not matter, in this specification, whether *Streptococci* is sensitive to conventional antibiotics or resistant to conventional antibiotics. In other words, it does not matter whether *Streptococci* acquires resistance to conventional antibiotics.

The term "treatment" or "treatment", as used herein, refers to all actions which suppress infections caused by *Streptococci,* and alleviate pathological conditions of diseases caused by *Streptococci.*

To increase efficiency for this purpose, antibacterial substances, which can provide antibacterial activity against other bacterial species, can be added to the pharmaceutical composition of the present disclosure.

A pharmaceutically acceptable carrier included in the pharmaceutical composition of the present disclosure is one commonly used in preparation. The pharmaceutically acceptable carrier may include any one selected from the group consisting of lactose, dextrose, sucrose, sorbitol, mannitol, starch, acacia gum, calcium phosphate, alginate, gelatin, calcium silicate, microcrystalline cellulose, polyvinylpyrrolidone, cellulose, water, syrup, methyl cellulose, methyl hydroxybenzoate, propyl hydroxybenzoate, talc, magnesium stearate, and mineral oil, but is not limited thereto. In addition to the ingredients, the pharmaceutical composition of the present disclosure may further include any one selected from the group consisting of lubricants, wetting agents, sweeteners, flavoring agents, emulsifiers, suspending agents, and preservatives.

The pharmaceutical composition of the present disclosure may be administered through oral administration or parenteral administration. In the case of parenteral administration, the pharmaceutical composition may be administered using intravenous administration, intraperitoneal administration, intramuscular administration, subcutaneous administration, or local administration. In addition, application or spraying to the affected area may also be used, but the administration method is not limited thereto.

The pharmaceutical composition of the present disclosure may be formulated using the pharmaceutically acceptable carrier and/or an excipient by a method, which can be easily performed by those skilled in the art to which the present disclosure pertains. Thereby, the pharmaceutical composition of the present disclosure may be prepared in unit dosage form or may be prepared by placing the pharmaceutical composition in a multi-dose container. In this case, the formulation of the pharmaceutical composition may be in the form of a solution, suspension, or emulsion in an oil or aqueous medium or may be in the form of an extract, a powder, a granule, a tablet, or a capsule. In the formulation, a dispersant or stabilizer may be further included.

In addition, suitable application, spraying, and dosage of the pharmaceutical composition vary depending on factors such as formulation method, administration method, age, weight, sex, severity of disease symptoms, food, administration time, administration route, excretion rate, and reaction sensitivity. Usually, a skilled doctor or veterinarian can easily determine and prescribe an effective dosage for the desired treatment.

The pharmaceutical composition of the present disclosure may be developed as an antibiotic, a disinfectant, a sterilizer, and a therapeutic agent.

### Advantageous Effects

A method of treating infections caused by *Streptococci,* by using a pharmaceutical composition containing an antibacterial protein SBL2200 as an active ingredient, in which the antibacterial protein SBL2200 contains an amino acid sequence represented by SEQ ID NO: 1 according to the present disclosure, can also be effective against *Streptococci* which have acquired resistance to conventional antibiotics or antibacterial substances. On the other hand, the antibacterial protein SBL2200 of the present disclosure specific for *Streptococci* does not affect normal flora other than *Streptococci,* so it can minimize side effects resulting from the use of the pharmaceutical composition containing the antibacterial protein SBL2200 as an active ingredient. Meanwhile, conventional antibiotics have the disadvantages of harming beneficial bacteria and causing various side effects.

### Description of Drawings

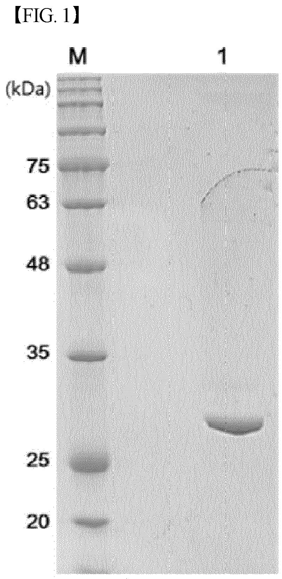

FIG. 1 shows an electrophoresis photograph showing the results of preparing an antibacterial protein SBL2200 specific for *Streptococci* in the form of a recombinant protein, the antibacterial protein SBL2200 containing an amino acid sequence represented by SEQ ID NO: 1, in which lane M represents a protein size marker, and lane 1 represents a chromatography flow through during secondary purification.

### Best Mode

Hereinafter, the present disclosure will be described in more detail based on examples, but these examples are only illustrative of the present disclosure, and the scope of the present disclosure is not limited to these examples.

### Example 1: Preparation of Streptococci-specific Antibacterial Protein SBL2200

Preparation of an antibacterial protein SBL2200 specific for *Streptococci,* will be described below. In this example, *Escherichia coli* AB-22 was used as a production strain, constructed by the present inventor(s) by transforming *E. coli* with a plasmid containing a nucleic acid sequence represented by SEQ ID NO. 2.

20 µL of *Escherichia coli* AB-22 was inoculated into 20 mL of LB medium (Tryptone 10 g/L, yeast extract 5 g/L, and NaCl 10 g/L) supplemented with 50 µg/mL kanamycin. Afterward, the LB medium was cultured with shaking at a temperature of 37°C overnight. The next day, the overnight cultured medium was added at a volume ratio of 1/100 to 1 L of LB medium supplemented with 50 µg/mL kanamycin. Culture was performed at a temperature of 37°C with a stirring speed of 200 rpm. When the cell concentration reached 0.3 to 0.35 based on absorbance at a wavelength of 600 nm, the culture temperature was changed to 25°C. After about 20 minutes, when the cell concentration reached 0.5 based on absorbance at a wavelength of 600 nm, L-arabinose was added so that the final concentration was 0.2%. Thereby, expression of an antibacterial protein SBL2200 containing an amino acid sequence represented by SEQ ID NO: 1 was induced. Thereafter, an additional 6-hour culture was performed.

After completion of the culture, the cell culture broth was taken and centrifuged at a temperature of 4°C for 10 minutes at 6,000 rpm to recover the cell pellet. The recovered cell pellet was suspended in 40 mL of a buffer (50 mM Potassium phosphate, 2 M NaCl, and pH 7.5). The cells of the cell suspension prepared in this way were disrupted using sonication. The sonication was performed by applying ultrasonic waves for 3 seconds to break the cells, stopping for 3 seconds. This process was repeated for a total of 15 minutes and in an ice bath.

After the cell disruption, the cell lysate was centrifuged at 13,000 rpm for 20 minutes at a temperature of 4°C, and the obtained supernatant was subjected to conventional hydrophobic interaction chromatography and anion-exchange chromatography purification processes.

The purification process is briefly explained as follows. In this example, 5 mL of HiTrap^{™} Phenyl HP (GE Healthcare) was used as a hydrophobic interaction resin, and 5 mL of HiTrap^{™} Q HP (GE Healthcare) was used as an anion-exchange resin. As a first purification process, hydrophobic interaction chromatography was performed after pre-equilibrating a column with a buffer A (50 mM Potassium phosphate, 2 M NaCl, and pH 7.5). A sample was loaded onto the column. Thereafter, washing was performed by flowing 5 CV (Column Volume) of the buffer A at a flow rate of 5 mL/min. After the washing, hydrophobic interaction chromatography, which was the first purification process, was performed under the following condition that a concentration gradient from the buffer A to the buffer B (50 mM Potassium phosphate and pH 7.5) was changed from 0% to 100% at a flow rate of 5 mL/min. Next, anion-exchange chromatography, which was a second purification process, was performed using the obtained first purification eluate. The anion-exchange chromatography was performed after pre-equilibrating a column with the buffer B (50 mM Potassium phosphate and pH 7.5). The sample obtained during the first purification process was loaded onto the column. Thereafter, washing was performed by flowing 10 CV under the following condition that the concentration gradient from the buffer B to a buffer C (50 mM Potassium phosphate, 1 M NaCl, and pH 7.5) was 30% at a flow rate of 5 mL/min. After the washing, chromatography was performed at a flow rate of 5 mL/min under following condition that the concentration gradient from the buffer B to the buffer C was 30% to 100%. In this process, elution of the antibacterial protein SBL2200 containing the desired amino acid sequence represented by SEQ ID NO: 1 was achieved. FIG. 1 shows the results of electrophoresis analysis of the antibacterial protein SBL2200 purified using a chromatographic purification process.

Among the obtained purified fractions, those containing high concentrations of the antibacterial protein SBL2200 were collected. The collected fractions were dialyzed against a buffer (50 mM Potassium phosphate and pH 7.5) to perform medium exchange. Through this, it was possible to secure the antibacterial protein SBL2200 solution with a purity of 90% or more.

Buffer exchange was performed for this concentrated SBL2200 solution with a buffer (50 mM Potassium phosphate, 5% (w/v) Sorbitol, 0.1%(w/v) poloxamer 188 and pH 7.5). Thereby, "an SBL2200 pharmaceutical composition" was prepared.

### Example 2: Investigation of Antibacterial Activity of Antibacterial Protein SBL2200

Antibacterial activity of the antibacterial protein SBL2200 specific for *Streptococci,* and prepared according to Example 1 was examined. Details of test strains subject to antibacterial activity investigation are shown in Table 1.

**[Table 1]**

| Test Strains Subject to Antibacterial Activity Investigation | | |
|---|---|---|
| Species | Strain | Source |
| *Streptococcus agalactiae* | ATCC 27956 | ATCC |
| | CCARM 4523 | CCARM |
| | CCARM 4537 | |
| *Streptococcus pyogenes* | CCARM 4528 | CCARM |
| | CCARM 4533 | |
| *Staphylococcus aureus* | ATCC 33591 | ATCC |
| | CCARM 3090 | CCARM |
| *Acinetobacter baumannii* | CCARM 12202 | CCARM |
| *Clostridioides difficile* | CCARM 0186 | CCARM |
| *Pseudomonas aeruginosa* | ATCC BAA-47 | ATCC |
| *Klebsiella pneumoniae* | CCARM 10332 | CCARM |
| *Escherichia coli* | CCARM 1G938 | CCARM |
| CCARM: Culture Collection of Antimicrobial Resistance Microbes; | | |
| ATCC: American Type Culture Collection | | |

A turbidity reduction assay and MIC (Minimum Inhibitory Concentration) test were used to investigate antibacterial activity. The turbidity reduction assay was conducted as follows. Test bacteria were suspended in physiological saline so that absorbance of the prepared cell suspension was about 0.7 at a wavelength of 600 nm. Thereafter, 0.1 mL of antibacterial protein SBL2200 solution was added (final concentration: 2.5 µM) to 0.9 mL of this suspension. Then, the absorbance was measured at a wavelength of 600 nm for 30 minutes. For preparing a negative control, a buffer (50 mM Potassium phosphate, 5% (w/v) Sorbitol, 0.1%(w/v) poloxamer 188, and pH 7.5) without the antibacterial protein SBL2200 was used instead of the antibacterial protein SBL2200 solution. An MIC was determined by conducting a routine MIC test in accordance with CLSI guidelines.

As a result, the antibacterial protein SBL2200 showed lytic activity only against *Streptococci,* meanwhile, the antibacterial protein SBL2200 did not show lytic activity against other test bacterial strains. The results are presented in Table 2.

**[Table 2]**

| Antibacterial Activity Investigation Results | | | |
|---|---|---|---|
| Species | Strain | Susceptibility | MIC (µg/ml) |
| *Streptococcus agalactiae* | ATCC 27956 | Susceptible | 6.25 |
| | CCARM 4523 | Susceptible | 6.25 |
| | CCARM 4537 | Susceptible | 6.25 |
| *Streptococcus pyogenes* | CCARM 4528 | Susceptible | 1.56 |
| | CCARM 4533 | Susceptible | 1.56 |
| *Staphylococcus aureus* | ATCC 33591 | Insusceptible | - |
| | CCARM 3090 | Insusceptible | - |
| *Acinetobacter baumannii* | CCARM 12202 | Insusceptible | - |
| *Clostridioides difficile* | CCARM 0186 | Insusceptible | - |
| *Pseudomonas aeruginosa* | ATCC BAA-47 | Insusceptible | - |
| *Klebsiella pneumoniae* | CCARM 10332 | Insusceptible | - |
| *Escherichia coli* | CCARM 1G938 | Insusceptible | - |

From these results, it was confirmed that the antibacterial activity of the antibacterial protein SBL2200 of the present disclosure was significantly specific for *Streptococci.*

From the results, it was confirmed that the antibacterial protein SBL2200 specific for *Streptococci* of the present disclosure could lyse *Streptococci,* thereby ultimately contributing to killing *Streptococci.* These antibacterial properties of the antibacterial protein SBL2200 showed that the pharmaceutical composition containing the antibacterial protein SBL2200 specific for *Streptococci* could be used for the purpose of killing *Streptococci* when infection caused by *Streptococci* occurred. In addition, the antibacterial properties of the antibacterial protein SBL2200 showed that the pharmaceutical composition containing the antibacterial protein SBL2200 could also be used in the same way as conventional antibiotics for the purpose of treating infections caused by *Streptococci.*

### Example 3: Investigation of Therapeutic Effects of Antibacterial Protein SBL2200 Specific for Streptococci on streptococcal Infection

Therapeutic effects of the antibacterial protein SBL2200 specific for *Streptococci* on *streptococcal* infection were investigated through an infection animal model test. Specifically, 5-week-old ICR mice [specific pathogen-free (SPF) grade] weighing approximately 20 g were used as test animals. A total of 20 animals were divided into 2 groups (10 animals per group). Then, infections were induced by intravenously administering *Streptococcus agalactiae* CCARM 4503 at a concentration of 1× 10⁸ CFU per mouse (i.e., 1× 10⁸ CFU/mouse). For the treatment group, 0.2 mL of the pharmaceutical composition containing the antimicrobial protein SBL2200 was intravenously administered at 30 minutes, 12 hours, and 24 hours after the forced bacterial infections. For the control group, only a buffer solution (50 mM potassium phosphate, 5% (w/v) sorbitol, 0.1% (w/v) poloxamer 188, and pH 7.5) was administered in the same volume. The buffer was administered at the same time points as the administration of the pharmaceutical composition containing the antimicrobial protein SBL2200, specifically at 30 minutes, 12 hours, and 24 hours after the forced bacterial infections. The number of dead animals was recorded daily for 5 days following the forced bacterial infections, and the occurrence of any specific abnormalities was observed twice a day, in the morning and afternoon.

With the results, clear treatment effects were confirmed. The number of dead animals is shown in Table 3 below. It was confirmed that the administration of the pharmaceutical composition of the antibacterial protein SBL2200 of the present disclosure resulted in a significant improvement in the survival rate of infected animals. Additionally, in the control group, various specific reactions such as erythema of lid margin and decreased activity were observed. Unlike the control group, no such specific reaction was observed in the animals in the treatment group administered the pharmaceutical composition of antibacterial protein SBL2200.

**[Table 3]**

| Streptococcal Infection Treatment Effects | | | | | | | |
|---|---|---|---|---|---|---|---|
| Group | Number of Dead Animals | | | | | Number of Dead Animals/Number of Test Animals | Mortality rate (%) |
| | Days after Forced Infections with Bacteria | | | | | | |
| | 1 | 2 | 3 | 4 | 5 | | |
| Control | 0 | 3 | 1 | 1 | 0 | 5/10 | 50 |
| Treatment | 0 | 0 | 0 | 0 | 0 | 0/10 | 0 |

From the results, it was confirmed that the antibacterial protein SBL2200 of the present disclosure was effective in treating streptococcal infections. These properties showed that the pharmaceutical composition containing the antibacterial protein SBL2200 as an active ingredient could be used to treat streptococcal infections.

The specific parts of the present disclosure have been described in detail above. For those skilled in the art, these specific techniques are merely examples of preferred implementations. It is clear that the scope of the present disclosure is not limited by these examples. Accordingly, the actual scope of the present disclosure will be defined by the appended claims and their equivalents.
Depository Institution Name: KCTC
Accession Number: KCTC15033BP
Deposit Date: 20220712

### BUDAPEST TREATY ON THE INTERNATIONAL RECOGNITION OF THE DEPOSIT OF MICROORGANISMS FOR THE PURPOSE OF PATENT PROCEDURE INTERNATIONAL FORM

### RECEIPT IN THE CASE OF AN ORIGINAL DEPOSIT

### Issued pursuant to Rule 7.1

To: iNtRON Biotechnology
Address: 137, Sagimakgol-ro, Jungwon-gu, Seongnam-si, Gyeonggi-do, Republic of Korea

| **I.** IDENTIFICATION OF THE MICROORGANISM | |
|---|---|
| Identification reference given by the DEPOSITOR: | Accession number given by the INTERNATIONAL DEPOSITARY AUTHORITY: |
| ***Escherichia coli* AB-22** | |
| | **KCTC 15033BP** |

| **II.** SCIENTIFIC DESCRIPTION AND/OR PROPOSED TAXONOMIC DESIGNATION | |
|---|---|
| The microorganism identified under I above was accompanied by: | |
| [ ] a scientific description | |
| [ ] a proposed taxonomic designation | |
| (Mark with a cross where applicable) | |

| **III.** RECEIPT AND ACCEPTANCE | |
|---|---|
| This International Depositary Authority accepts the microorganism identified under **I** above, which was received by it on **July 12, 2022.** | |

| **IV.** RECEIPT OF REQUEST FOR CONVERSION | |
|---|---|
| This microorganism identified under **I** above was received by the International Depositary Authority on and a request to convert the original deposit to a deposit under the Budapest Treaty was received by it on. | |

| **V.** INTERNATIONAL DEPOSITARY AUTHORITY | |
|---|---|
| Name: Korean Collection for Type Cultures | Signature(s) of person(s) having the power to represent the International Depositary Authority or of authorized official(s): |
| Address: 181, Ipsin-gil, Jeongeup-si, Jeonbuk-do, Republic of Korea (56212) | |
| | Representative |
| | Date: **July 12, 2022** |

## Claims

1. An antibacterial protein SBL2200 with an amino acid sequence represented by SEQ ID NO: 1, which has streptococci-specific antibacterial activity

2. The antibacterial protein of Claim 1, the antibacterial protein SBL2200 is made of 279 amino acids and has a molecular weight of 31.3 kDa.

3. A pharmaceutical composition for treating infections caused by *Streptococci,* the pharmaceutical composition comprising the antibacterial protein SBL2200 of claim 1 as an active ingredient.

4. The pharmaceutical composition of Claim 3, wherein the pharmaceutical composition is used as an antibiotic, a disinfectant, a sterilizer, and a therapeutic agent.

5. A method of preparing the antibacterial protein SBL2200 of claim 1 by using a production strain *Escherichia coli* AB-22 (accession number KCTC15033BP) constructed by transforming *E. coli* with a plasmid containing a nucleic acid sequence represented by SEQ ID NO: 2.
